# EUROPEAN PATENT APPLICATION

(11) **EP 4 332 975 A1**
(43) Date of publication of application: **06.03.2024**
(21) Application number: 22192531.6
(22) Date of filing: 29.08.2022
(51) Int. Cl.: G16B 30/10, C12Q 1/6869

(54) **METHOD FOR ELIMINATING NON-NATURAL SEQUENCE PORTIONS FROM FASTQ SEQUENCE DATA**

(71) Applicant: Siemens Healthcare Diagnostics Products GmbH, 35041 Marburg (DE)
(72) Inventor: Kauppila, Timo, 50823 Köln (DE); Lenz, Florian, 50931 Köln (DE); Zacherle, Tobias, 50668 Köln (DE)
(74) Representative: Siemens Healthineers Patent Attorneys

(57) **Abstract**

The present invention relates to a method for eliminating non-natural nucleic acid sequence portions from paired-end reads of nucleic acid fragments comprising the steps of (a) providing paired-end reads of nucleic acid fragments, wherein one of the two paired-end reads that constitute a read pair of a nucleic acid fragment is converted into its reverse complement form; (b) aligning the two paired-end reads of the read pair of step (a) with each other; (c) identifying overlapping sequence regions in the aligned paired-end reads; (d) identifying a unique molecular identifier (UMI) sequence as non-natural nucleic acid sequence in the aligned paired-end reads; (e) optionally storing said identified UMI sequence; (f) deleting said identified UMI sequence, if present, at the 5' end of each paired-end read; and (g) deleting said identified UMI sequence, if present, at the 3' end of each paired-end read.

## Description

### TECHNICAL FIELD

The present invention lies in the field of nucleic acid sequencing data processing and relates to a method for eliminating non-natural nucleic acid sequence portions from paired-end reads of nucleic acid fragments comprising the steps of (a) providing paired-end reads of nucleic acid fragments, wherein one of the two paired-end reads that constitute a read pair of a nucleic acid fragment is converted into its reverse complement form; (b) aligning the two paired-end reads of a read pair of step (a) with each other; (c) identifying overlapping sequence regions in thealigned paired-end reads; (d) identifying a unique molecular identifier (UMI) sequence as non-natural nucleic acid sequence in the aligned paired-end reads; (e) optionally storing said identified UMI sequence; (f) deleting said identified UMI sequence, if present, at the 5' end of each paired-end read; and (g) deleting said identified UMI sequence, if present, at the 3' end of each paired-end read.

### BACKGROUND

NGS (Next Generation Sequencing) data are typically transformed into the Fastq format (Cock, P.J.A. et al., Nucleic Acids Research, Vol. 38 (6), 1767 (2010)) to allow alignment of sequencing reads to a reference genome. The bases and quality scores of each read are stored in this text-based data format. In the case of paired-end sequencing, i.e. when DNA fragments are sequenced from both ends, there are two Fastq files, one where first read (R1) information is stored and another one where second read (R2) information is stored for the respective DNA fragment.

Fastq files of high data quality are becoming increasingly important since they are used as input for alignment programs and data analysis. For this reason, sequence contaminations that are not present on the original DNA fragments must be eliminated. Possible artificial contaminations are, among others, Unique Molecular Identifiers (UMIs), i.e. short nucleotide sequences, typically consisting of 3 to 12 nucleotides, that are attached to the original DNA fragment prior to PCR amplification in some sequencing techniques and that help to attribute PCR duplicates later back to an original DNA fragment. UMIs are typically ligated to the termini of the DNA fragments, so that they make up the first sequenced nucleotides when sequenced and they can, in certain scenarios, be among the last nucleotides sequenced, e.g. when the read is long enough and reaches over the 3' end of the DNA fragment of interest. These nucleotides do typically not provide any useful information about the DNA in the original sample but are a purely technical contamination whose presence during reference sequence alignment may lead to misinterpretations.

A further class of sequence contaminations are sequencing adapters, typically consisting of 30 to 40 nucleotides, which are typically ligated to both ends of the original DNA fragments in order to attach necessary information such as demultiplexing indices to the fragments and to be able to anchor the fragments on the flow cell of the sequencer. If the fixed read length for the sequencing read is longer than the original length of the DNA fragment not only the biologically relevant nucleotides are stored in a read, but also the first nucleotides of a sequencing adapter at the 3' end of the read. Similar to the UMIs, these adapter nucleotides do not provide biologically or technically expedient information.

Thus, UMIs and sequencing adaptors, which are both non-natural nucleic acid sequence portions, should be removed from the Fastq reads before alignment, as they might disturb subsequent alignment processes.

There is hence a need for a robust and reliable method to remove non-natural nucleic acid sequence portions from paired-end reads of nucleic acid fragments. In particular, it is necessary to remove all occurring UMI sequences, be it 5' or 3' from the read sequences.

### SUMMARY

The present invention addresses this need and presents a method for eliminating non-natural nucleic acid sequence portions from paired-end reads of nucleic acid fragments comprising the steps of (a) providing paired-end reads of nucleic acid fragments, wherein one of the two paired-end reads that constitute a read pair of a nucleic acid fragment is converted into its reverse complement form; (b) aligning the two paired-end reads of a read pair of step (a) with each other; (c) identifying overlapping sequence regions in thealigned paired-end reads; (d) identifying a unique molecular identifier (UMI) sequence as non-natural nucleic acid sequence in the aligned paired-end reads; (e) optionally storing said identified UMI sequence; (f) deleting said identified UMI sequence, if present, at the 5' end of each paired-end read; and (g) deleting said identified UMI sequence, if present, at the 3' end of each paired-end read.

The provided method advantageously allows to remove 3' UMI sequence contaminations which current tools and methods do not consider. The removal of these artificial nucleotide sequences yields higher quality Fastq reads and better alignment/variant calling downstream.

In a preferred embodiment said identification of the overlap in the aligned paired-end reads comprises the steps of: (i) sliding one paired-end read past the other paired-end read over the entire length of the paired-end reads and determining for each position whether an overlap exists; and (ii) selecting the sliding position which provides the maximum number of overlapping bases.

In a further preferred embodiment said step (ii) of selecting the sliding position of the maximum number of overlapping bases is performed only if the overall number of mismatches between the paired-end reads is below 5% to 15% of all bases.

In yet another preferred embodiment said step (ii) of selecting the sliding position of the maximum number of overlapping bases is performed only if the likelihood of correct overlapping (i.e. not purely by chance) is above 90%, 95%, or 99%.

In a further preferred embodiment of the method according to the invention said step (ii) of selecting the sliding position of the maximum number of overlapping bases is performed only if a minimum number of overlapping bases is reached, wherein said minimum number is 10, 15, 20, 25 or 30.

In a preferred embodiment said identification of UMI sequences in the aligned paired-end reads uses recorded information on said UMI sequences and/or standardized or conventional position and length information.

It is particularly preferred that said identification of UMI sequences is an identification of a 3' UMI sequence in one paired-end read and wherein said identification requires the identification of an overlapping 5' UMI sequence in the other paired-end read of a read pair.

In an additional preferred embodiment the method according to the invention comprises as additional step (d-1) identifying an adaptor sequence as non-natural nucleic acid sequence, if present, in the aligned paired-end reads; and as step (f-1) deleting said identified adaptor sequence at the 3' end of each paired-end read. Said adaptor sequence is typically identified in the 3'-terminus of each paired-end read of the aligned paired-end reads.

In a further preferred embodiment said adaptor sequence is identified as being located 3' of an UMI sequence and wherein both paired-end reads of the read pair comprise UMI sequences completely overlapping in the aligned paired-end reads.

In further preferred embodiments said deletion of said identified adaptor sequence is performed concomitantly with the deletion of said 3' UMI sequence.

It is particularly preferred that each paired-end read of a read pair has a length of 10 to 10.000 bases.

In a further aspect the present invention relates to an in vitro method for diagnosing a subject, comprising the steps: (a) performing a massively parallel nucleic acid sequencing of nucleic acids extracted from a subject's sample, preferably a tumor biopsy sample or a liquid biopsy sample, to obtain paired-end reads, wherein one of the two paired-end reads that constitute a read pair of a nucleic acid fragment is converted into its reverse complement form; (b) aligning the two paired-end reads of a read pair obtained in step (a) with each other; (c) identifying overlapping sequence regions in the aligned paired-end reads; (d) identifying a unique molecular identifier (UMI) sequence as non-natural nucleic acid sequence and optionally an adaptor sequence as non-natural nucleic acid sequence, if present, in the aligned paired-end reads; (e) deleting said identified UMI sequence, if present, at the 5' end of each read, and optionally, if present, also at the 3' end of each paired-end read; (f) optionally deleting said identified adaptor sequence, if present, at the 3' end of each paired-end read; (g) inputting a truncated read obtained in step (e) and optionally in step (f) into a genomic sequence alignment in order to detect sequence differences vis-à-vis a reference sequence; (h) comparing identified sequence differences with a reference library of sequence differences linked to associated diseases; and (i) deducing the subject's health status and prognosis from the comparison result obtained in step (h).

The deletion of said non-natural nucleic acid sequences in the original paired-end reads in steps (e) and (f) and use of the obtained truncated reads in subsequent sequence data processing increases the accuracy of sequence data analysis, such as the comparison of identified sequence differences with a reference library of sequence differences.

In a preferred embodiment of the vitro method for diagnosing a subject, the method further comprises providing a report in electronic, web-based, or paper form, to a subject or to another person or entity, a caregiver, a physician, an oncologist, a hospital, a clinic, a third-party payor, an insurance company or a government office.

In a further preferred embodiment the report comprises one or more of: (i) output from the method, comprising the determined sequence difference, if present; (ii) information on the meaning of the comparison results wherein said information comprises information on prognosis and/or potential or suggested therapeutic options; (iii) information on the likely effectiveness of a therapeutic option, the acceptability of a therapeutic option, or the advisability of applying the therapeutic option to a subject having a sequence modification; or (iv) information, or a recommendation on the administration of a drug, the administration at a preselected dosage, or in a preselected treatment regimen, in combination with other drugs, to the subject.

### BRIEF DESCRIPTION OF THE DRAWINGS

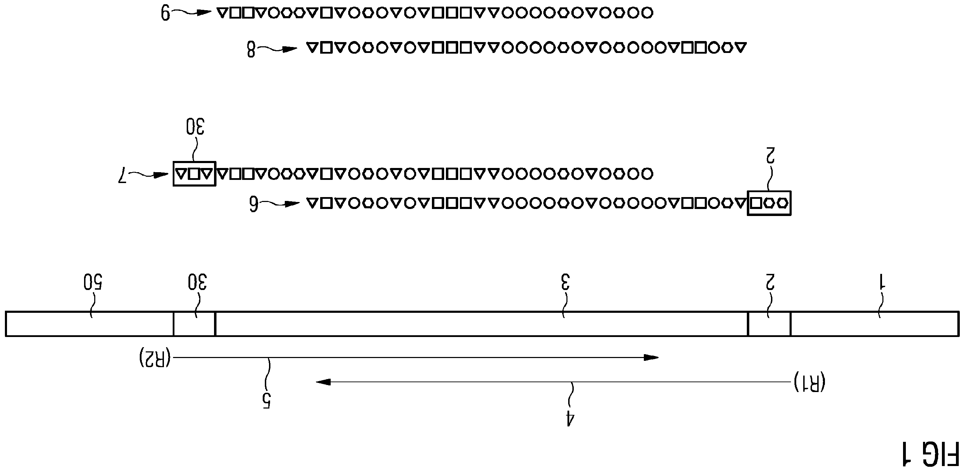

FIG. 1 shows an exemplary embodiment of the invention. The figure depicts two symbolic paired-end sequence reads R1 (4) and R2 (5) which constitute a read pair of a nucleic acid fragment. Below these reads a DNA molecule is shown which comprises at the 5' end and at the 3' end an adaptor sequence (1 and 50), a first UMI sequence (2), an original DNA fragment sequence (**3**) and a second UMI sequence (**30**). The sequence of read R1 (**4, 6**) comprises the first UMI sequence (**2**), but not the second UMI sequence (**30**). Likewise, the sequence of read R2 (**5**, **7**) comprises the second UMI sequence (**30**), but not the first UMI sequence (**2**). The first and second UMI sequences (**2** and **30**) are depicted as boxes in the aligned read sequences (**6** and **7**). It is noted that the sequence of R2 (**5**, **7**) is presented as reverse complement for the sake of comparability of the sequences. After identification of the UMI sequences they are eliminated from the original reads and truncated reads (**8** and **9**) are provided. This elimination step requires a truncation only at the 5' end of each original read. The two truncated reads (**8** and **9**) are overlaid in a way that corresponding bases are at the same position (after finding the maximal/best overlap). In this embodiment, none of the original paired-end reads covers the entire original DNA fragment and therefore the overlap does not cover the entire original DNA fragment sequence (**3**). Consequently, only a first UMI sequence (**2**) and a second UMI sequence **(30)** need to be removed, yet no sequences at the opposite termini of the original reads have to be eliminated. The figure shows DNA bases as symbols.

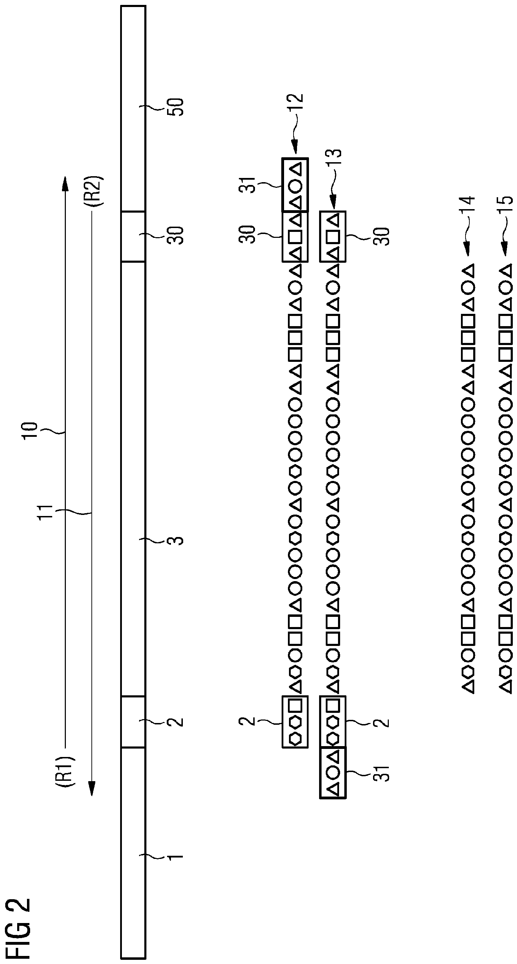

**FIG 2** shows another exemplary embodiment of the invention. The figure depicts two symbolic paired-end sequence reads R1 **(10)** and R2 **(11)** which constitute a read pair. Below these reads a DNA molecule is shown which comprises at the 5' end and at the 3' end an adaptor sequence (**1** and **50**), a first UMI sequence **(2),** the original DNA fragment sequence **(3)** and a second UMI sequence (**30**). The sequence of read R1 **(10, 12)** comprises the first UMI sequence **(2)** and the second UMI sequence **(30),** as well as a part of the adaptor sequence **(50, 31).** Likewise, the sequence of read R2 **(11, 13)** comprises the second UMI sequence **(30),** the first UMI sequence **(2)** and a part of the adaptor sequence (1, **31**). The first and second UMI sequences (**2** and **30**) are depicted as boxes in the aligned read sequences (**12** and **13**). The partial adaptor sequences are depicted as bold boxes **(31)** in the aligned read sequences **(12** and **13**). It is noted that the sequence of R2 **(11, 13)** is presented as reverse complement for the sake of comparability of the sequences. After identification of the UMI and adaptor sequences they are eliminated from the original reads and truncated reads **(14** and **15)** are provided. This elimination step requires a truncation at both ends of the original reads, i.e. at the 5' and 3' end of each read. The depicted reads **(14** and **15)** are overlaid in a way that corresponding bases are at the same position (after finding the maximal/best overlap). In this example the overlap covers the entire original DNA fragment sequence (3) and therefore a first UMI sequence (2) and a second UMI sequence (30) are removed at both termini of both reads, as well as partial adaptor sequences (31). The figure shows DNA bases as symbols.

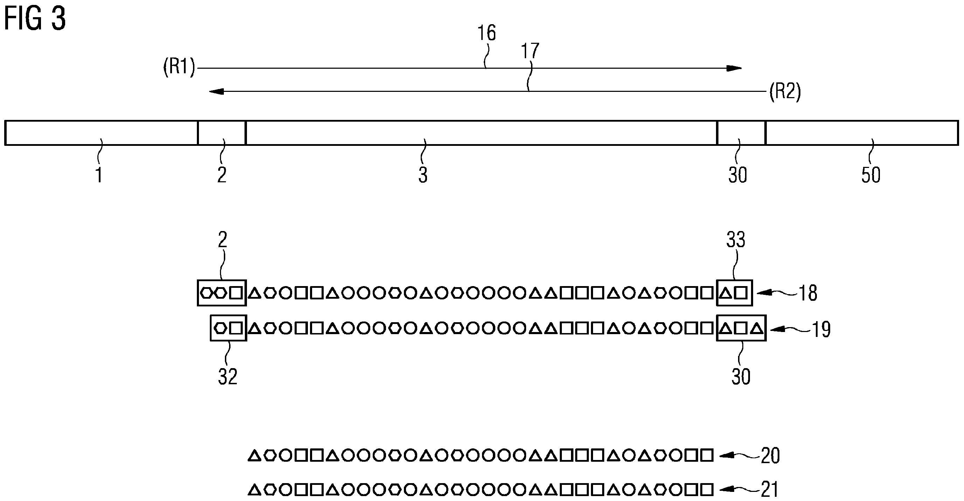

FIG 3 shows a further alternative embodiment according to the invention. The figure depicts two symbolic paired-end sequence reads R1 **(16)** and R2 **(17)** which constitute a read pair. Below these reads a DNA molecule is shown which comprises at the 5' end and at the 3' end an adaptor sequence **(1** and **50),** a first UMI sequence **(2),** the original DNA fragment sequence **(3)** and a second UMI sequence **(30).** The sequence of read R1 **(16, 18)** comprises the first UMI sequence **(2)** and a part of the second UMI sequence **(33).** The sequence of read R2 **(17, 19)** comprises the second UMI sequence **(30)** and a part of the first UMI sequence (**32**). The first and second UMI sequences **(2, 30, 32** and **33)** are depicted as boxes in aligned read sequences **(18** and **19**). It is noted that the sequence of R2 **(5, 7)** is presented as reverse complement for the sake of comparability of the sequences. After identification of the UMI sequences they are eliminated from the original reads and truncated reads **(20** and **21)** are provided. This elimination step requires a truncation at both ends of the reads, i.e. at the 5' and 3' end of each read. In this embodiment both reads **(20** and **21)** are overlaid in a way that corresponding bases are at the same position (after finding the maximal/best overlap). In this example, each of the original paired-end reads covers the entire original DNA fragment and therefore the overlap covers the entire original DNA fragment sequence (**3**). However, due to the absence of adaptor sequences only UMI sequences **(2, 30, 32** and **33)** are removed. The figure shows DNA bases as symbols.

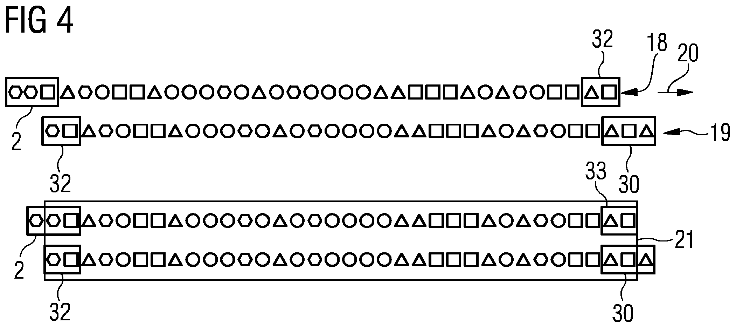

**FIG 4** shows the process of aligning two paired-end reads of a read pair and finding an overlap. Read R1 **(18)** is slided **(100)** over read R2 **(19)** until a suitable overlap has been reached. In the overlap a first UMI sequence **(2),** a second UMI sequence **(30),** as well as a partial first UMI sequence **(32)** and partial second UMI sequence **(33)** can be identified. The figure shows DNA bases as symbols.

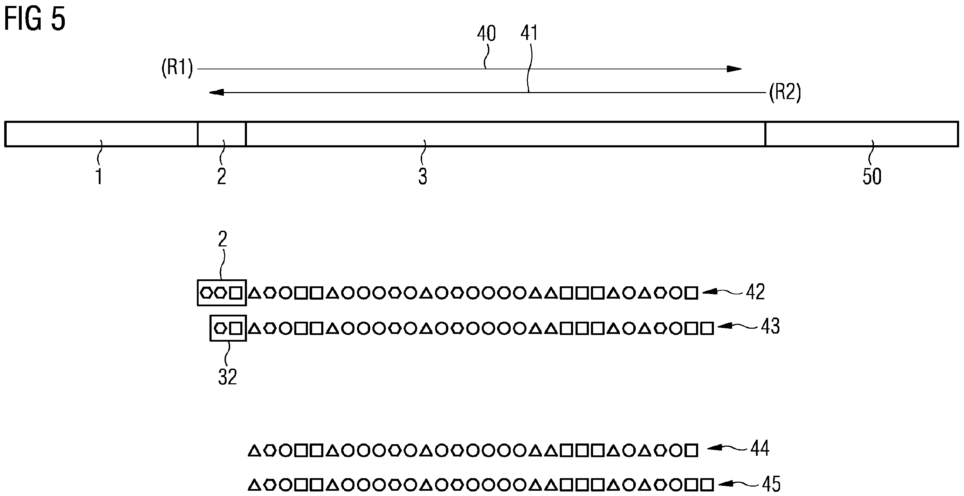

**FIG 5** shows a further exemplary embodiment of the invention. The figure depicts two symbolic paired-end sequence reads R1 **(40)** and R2 **(41)** which constitute a read pair. Below these reads a DNA molecule is shown which comprises at the 5' end and at the 3' end an adaptor sequence **(1** and **50),** a first UMI sequence **(2)** and the original DNA fragment sequence (**3**). The sequence of read R1 **(40, 42)** comprises the first UMI sequence **(2).** The sequence of read R2 **(41, 43)** comprises a part of the first UMI sequence **(32).** The first UMI sequences and parts of it **(2** and **32)** are depicted as boxes in aligned read sequences **(42** and **43**). After identification of the UMI sequences they are eliminated from the original reads and truncated reads **(44** and **45)** are provided. This elimination step requires a truncation at one end of each read. Both reads are overlaid in a way that corresponding bases are at the same position (after finding the maximal/best overlap). In this example the overlap does not cover the entire original DNA fragment sequence **(3).** Further, the reads comprise only one UMI sequence **(2).** Accordingly, only a first UMI sequence **(2)** and parts of it **(32)** are removed. The figure shows DNA bases as symbols.

### DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term "typically" indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms " (i) ", "(ii)", "(iii)" or "(a)", "(b)", "(c)", "(d)", or "first", "second", "third" etc. and the like in the description or in the claims, are used for distinguishing between similar elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e. the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary. It is also to be understood that the terminology used herein is for the purpose of describing particular embodiments only and is not intended to limit the scope of the present invention that will be limited only by the appended claims.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

As has been set out above, the present invention concerns in one aspect a method for eliminating non-natural nucleic acid sequence portions from paired-end reads of nucleic acid fragments comprising the steps of (a) providing paired-end reads of nucleic acid fragments, wherein one of the two paired-end reads that constitute a read pair of a nucleic acid fragment is converted into its reverse complement form; (b) aligning the two paired-end reads of a read pair of step (a) with each other; (c) identifying overlapping sequence regions in the aligned paired-end reads; (d) identifying a unique molecular identifier (UMI) sequence as non-natural nucleic acid sequence in the aligned paired-end reads; (e) optionally storing said identified UMI sequence; (f) deleting said identified UMI sequence, if present, at the 5' end of each paired-end read; and (g) deleting said identified UMI sequence, if present, at the 3' end of each paired-end read.

The term "sequence portion of paired-end reads of nucleic acid fragments" as used herein relates to nucleic acid sequence data, wherein the sequence data are obtained by any technique suitable to provide paired-end sequence data, typically in a high-throughput approach. The term "paired-end read" as used herein relates to nucleic acid sequence data of a sequence read originating from paired-end sequence data. The term "read pair" as used herein relates to a set of two paired-end reads which are sequenced from both ends of a single nucleic acid fragment. The reads are typically designated as read 1 (R1) and read 2 (R2). Due to the sequencing technology used, R1 and R2 are derived from opposite DNA strands of a single nucleic acid fragment. For this reason, when an overlap determination is to be performed, one of the read sequences has to be reverse complement. This sequence data may, for example, be obtained with next-generation sequencing (NGS) or second generation sequencing techniques. Corresponding sequencing approaches to obtain paired-end reads include any sequencing method that determines the nucleotide sequence of either individual nucleic acid molecules or expanded clones for individual nucleic acid molecules in a highly parallel fashion. For example, more than 10⁸ molecules may be sequenced simultaneously. The sequencing may be performed according to any suitable method capable of generating paired-end reads, e.g. as known to the skilled person. Typical platforms are short read sequencing platforms from manufacturers such as Illumina, Thermo Fisher Scientific (Ion Torrent), BGI, etc.

According to the presently described methodology any suitable sequencing read length may be used. It is preferred to make use of paired-end sequencing reads of a length of about 10 to about 10.000 bases, such as, e.g. 10, 100, 1000, or 10.000 or more nucleotides or any value in between the mentioned values. Most preferably, a length of 100 nucleotides is employed.

The term "UMI" or "unique molecular identifier" as used herein relates to a short non-natural nucleic acid sequence that is used to uniquely tag each nucleic acid fragment in a sample library. The UMI sequence is thus a unique barcode sequence which is added to each molecule in a given sample library, e.g. to be sequenced subsequently. By adding this barcode tag to each DNA fragment variant alleles can be counted in terms of mutations per fragment and PCR amplification induced errors can be eliminated. Thus, sensitivity and accuracy of variant detection is increased. The UMI sequence may have any suitable length, which may be adapted to the performed protocol. Typically, the UMI sequence has a length of 3 to 12 nucleotides. UMI sequences may further be present in combination with spacer or linker elements. UMI sequences may be present in the 5' region of a nucleic acid fragment, or on both termini of the fragment. Should UMI sequences be present at both termini of a fragment, the two UMI sequences are considered independent and usually differ in their sequence, although - by chance - might also have identical sequences.

The term "non-natural nucleic acid sequence" as used herein refers to an artificially generated sequence which is or was added to the biological DNA fragment, e.g. by means of ligation for technological purposes, like in the case of an adapter sequence which allows that the processed fragment attaches to the flow cell, or in the case of an UMI sequence, which is added to a biological DNA fragment to be able to identify, for instance, PCR duplicate reads.

In a first step of the method according to the invention, one of the paired-end reads of a read pair to be analysed is converted into its reverse complement form. In a typical embodiment, the read R2 may be converted in this way. This step allows for a base-to-base comparison for the identification of identical bases or stretches of bases. The "reverse complement form" or "reverse complement" of a sequence relates to the reverse and complementary sequence of the provided sequence, i.e. nucleotide A goes to T, G goes to C etc.

Subsequently, alignment of the two paired-end reads of a read pair after the conversion of one of the reads into a reverse complement form is performed. The terms "alignment" or "sequence alignment" or "aligning" as used herein generally relate to the process of sequence comparison and matching one sequence read with another sequence read. In the context of the present invention alignment exclusively relates to nucleotide sequences. For the performance of an alignment operation or sequence comparison any suitable algorithm or tool can be used.

The aligning step aims at the identification of overlapping sequence regions or bases in the aligned sequences. An "overlap" within the context of the present invention means that both paired-end reads as presented to the alignment operation comprise stretches of identical bases. Due to the sequencing methodology which starts from both termini of a nucleic acid fragment, an overlap is expected to be present at least in the 3' region of the paired-end reads. The overlap is accordingly expected within the original DNA fragment sequence (3) as depicted in FIG. 1 to 3 and 5.

In preferred embodiments the alignment and identification of the overlap of the two paired-end reads of a read pair comprises a step of sliding one read past another read over the entire length of the reads and determining for each position whether an overlap exists. The term "sliding" as used herein means that both sequences of the reads as described above, one being in reverse complement form, are compared in a base-by-base-like manner wherein one of the reads is moved into the direction where the terminal bases of the second read are present, thereby increasing the number of overlaid bases. Once an overlay of bases is given, a comparison operation is started to see how many overlaid bases are identical for a certain sliding position. Corresponding results are stored, e.g. in a computer program, computer memory or database. By repeating this step over the entire length of the paired-end reads, all sliding positions/overlay configurations can be compared with each other. Subsequently, the position in which the maximum number of matching bases in the overlap region was determined is selected as the most suitable sliding position indicating the best overlap of both paired-end reads.

In further preferred embodiments, the selection of the best overlap may be made dependent on certain conditions in order to avoid unspecific alignment results. It is accordingly preferred that the step of selecting the sliding position of the maximum number of matching bases is performed only if the overall number of mismatched bases in the overlap region of the analysed reads is below 5% to 15% of all bases in the overlap region. It is particularly preferred that the step of selecting the sliding position of the maximum number of overlapping bases is performed only if the overall number of mismatched bases in the overlap region between the analysed reads is below 5%, 6%, 7%, 8%, 9%, 10%, 11%, 12%, 13%, 14% or 15% of all bases in the overlap region, or any value in between the mentioned values. Higher numbers of mismatches may indicate sequencing errors or non-overlapping reads.

In further specific embodiments, the step of selecting the sliding position of the maximum number of matching bases is performed only if the likelihood of correct overlapping is above 90%, 95%, or 99%. This corresponds to low probability of overlap by chance (p-value). The term "likelihood of correct overlapping" as used herein means 1 minus the p value.

In a further specific set of embodiments, the step (ii) of selecting the sliding position of the maximum number of matching bases is performed only if a minimum number of overlapping bases of the reads is reached. The minimum number is preferably 10 bases. More preferably it is 15, 20, or 25 bases. In a particularly preferred embodiment, the minimum number is 30.

Once the overlap within the aligned paired-end reads of a read pair has been determined, an UMI sequence may be identified at one or both termini of the aligned paired-end reads. An UMI sequence may, for example, be present at the 5' end of read 1 (R1), e.g. as depicted in FIG. 1 (2). Similarly, an UMI sequence may be identified at the 5' end of read 2 (R2), e.g. as depicted in FIG. 1 (7). In specific embodiments, only one UMI sequence may be present, e.g. an UMI sequence at the 5' end of read 1 (R1) or read 2 (R2). In an alternative embodiment, both, 3'-UMI sequences and 5'-UMI sequences, may be identified, as e.g. depicted in FIG. 2 (2, 30) and FIG. 3 (33, 30), (2, 32).

The identification of an UMI sequence may be based on sequence information concerning UMI sequences present in the original sample library, e.g. the UMI sequence(s) used and/or its lengths and intended positions within the paired-end reads are known, thereby allowing for an identification for said UMI sequence(s). Preferably, the UMI sequences are sequences of known lengths at the 5' end of a read, possibly including a known number of "dark" bases, i.e. bases that are technically not part of the UMI sequence, but still have to be removed and trimmed as if they were part of the UMI sequence. So, for example for each read (R1 and R2) the first 5 bases can be automatically identified to be trimmed, if during library preparation UMI sequences of a length of 3 bases plus 2 adjacent dark bases are attached to both termini of the DNA fragment.

In a preferred embodiment, the identification of a 3' UMI sequence of one paired-end read requires the concomitant identification of an overlapping 5' UMI sequence of the other paired-end read in a read pair. As depicted in FIG. 2, on both reads 5' and 3' UMI sequences are present. By identifying an overlap of a 5' UMI sequence of one read and a 3' UMI sequence of the opposite read, both locations can be confirmed, thus allowing for a clear definition of the starting position of the 3' UMI sequence.

In a further preferred embodiment, the method additionally comprises as step (d-1) the identification of an adaptor sequence as non-natural nucleic acid sequence, if present, in the aligned paired-end reads of a read pair. The adaptor sequence may typically be located at a position 3' of the 3' UMI sequence, e.g. as depicted in FIG. 2 (31). The terms "adaptor" or "adaptor sequence" as used herein refer to a non-natural sequence, which has been ligated to the original DNA fragment during the preparation for sequencing. An adaptor may comprise binding sites for enzymes or index sequences etc. An adaptor sequence may only occur in sequencing reads which cover an original DNA fragment which is shorter than the maximum read sequencing length. In the context of the present invention, an adaptor may be identified as a sequence, which is located 3' of a 3' UMI sequence, which has been identified, e.g. by identifying an overlap of R1 and R2 reads as outlined above. The detection of an adaptor sequence advantageously requires the presence of a complete overlap of an UMI sequence at the same terminus. This complete overlap may be used for the identification of the start position of the adaptor sequence.

Once the UMI sequence and potentially the adaptor sequence is/are identified, the identified UMI sequence and optionally the identified adaptor sequence may be stored, e.g. in a computer program, computer memory or database. The storage is an optional step. The method may, in certain embodiments, also be performed without said storage step.

The stored UMI and/or adaptor sequence(s) may subsequently be used for retrieval, comparison, documentation or verification purposes. The UMI and/or adaptor sequence(s) may be stored together with an associated original DNA fragment sequence, preferably with all sequence items connected to the read pair associated with said UMI sequence. For example, information on the position where an alignment correspondence between one sequencing read and a further sequencing read was detected may be stored together with the UMI and/or adaptor sequence(s). For example, position information, information on the degree of correspondence, version and sequence identity information, base quality information of UMI bases (e.g. the Phred Score, which gives the error probability of the sequenced base encoded in ASCII code), etc. may be stored together with the UMI sequence information. In preferred embodiments, a format such as Fastq, BAM, SAM or CRAM may be used. BAM and SAM formats are designed to contain the same information. The SAM format is a human readable format and easier to process by conventional text based processing programs, such as, for example, standard Linux commands or python. The BAM format provides binary versions of the same data and is designed to provide a good compression rate. The CRAM format is similar to the BAM format. In this format the compression is driven by the reference the sequence data is aligned to.

In a subsequent step, the UMI sequence(s), and optionally the adaptor sequence(s), if present, may be deleted from the paired-end sequence reads in which it or they was/were identified. In one set of embodiments, the UMI sequence is present and has been identified only at one terminus of a paired-end read and is accordingly to be removed from said terminus only. For example, the UMI sequence may be present only at the 5' end of a paired-end read, typically of both paired-end reads of a read pair as depicted in FIG 1.

In a further set of embodiments, UMI sequences may be present and have been identified at both termini of a paired-end read, i.e. at the 5' and 3' end, e.g. as depicted in FIG. 2 (where the first UMI sequence (2) of R1 (12) represents the 5' UMI sequence of R1 and the second UMI sequence (30) of R1 (12) represents the 3' UMI sequence of R1; conversely, the second UMI sequence (30) of R2 (13) represents the 5' UMI sequence of R2, whereas the first UMI sequence (2) of R2 (13) represents the 3' UMI sequence of R2). The presence of such a second 3' UMI sequence may be detected in accordance with the above-mentioned overlap and UMI sequence identification step. In a further embodiment, in addition to UMI sequences at the 5' and 3' end, an adaptor sequence is identified at the 3' end, as outlined above.

The deletion of said UMI sequence(s) may be performed in a manner that all bases of an UMI sequence are deleted from the beginning of a paired-end read, if the UMI sequence is a 5' UMI sequence. Similarly, the deletion of said UMI sequence, and optionally of the adaptor sequence, may be performed in a manner that all bases which are located 3' of the UMI sequence including the UMI sequence itself, and optionally the adaptor sequence, are deleted if the UMI sequence is a 3' UMI sequence.

It is preferred that the deletion of the adaptor sequence is performed concomitantly with the deletion of the 3' UMI sequence.

Correspondingly edited sequence reads, i.e. truncated reads which do not contain UMI and adaptor sequences any longer because they have been deleted, may be stored in a computer program, a computer memory or database, e.g. in a format as mentioned above. These edited sequences which relate to the DNA fragment sequence of interest may subsequently be used for comparison purposes, e.g. for alignments with references sequences etc. Such an alignment typically comprises a process of sequence comparison and matching a sequencing read with a genomic sequence location.

The term "reference sequence" as used herein relates to a sequence, which is used for genomic alignment purposes within the context of the present invention. The reference sequence is typically a genomic sequence or part of a genomic sequence. In typical embodiments, the reference sequence is a human genomic sequence. In specific embodiments, the reference sequence may alternatively be a non-human genomic sequence such as monkey-, mouse-, rat-, bovine-sequence, a domestic animal sequence, a companion animal sequence etc.

The sequence may either be provided in a sense direction, or in a reverse-complement direction. The reference sequence may be selected as any suitable genomic sequence derivable from databases as known to the skilled person. For example, a reference sequence may be derived from the reference assembly provided by the Human Genome Reference Consortium. Also envisaged are further similar reference sequences. The reference sequence may further be limited to certain sectors of the genome, e.g. specific chromosomes, or parts of a chromosome, e.g. exons or certain genes, groups of genes or gene clusters etc. It is preferred that the reference sequence is a well established, curated and/or controlled sequence which comprises advantageously no or only a minimal proportion of sequencing errors. Information on the position where a genomic alignment correspondence between a sequencing read and a reference sequence was detected may be stored together with the sequence information. For example, genomic position information, information on the degree of correspondence, version and identity information on the reference sequence etc. may be stored together with the sequence information, e.g. in a format as mentioned herein above.

In a further aspect the present invention relates to an in vitro method for diagnosing a subject, comprising: (a) performing a massively parallel nucleic acid sequencing of nucleic acids extracted from a subject's sample, preferably a tumor biopsy sample or a liquid biopsy sample, to obtain paired-end reads, wherein one of the two paired-end reads that constitute a read pair of a nucleic acid fragment is converted into its reverse complement form; (b) aligning the two paired-end reads of a read pair obtained in step (a) with each other; (c) identifying overlapping sequence regions in the aligned paired-end reads; (d) identifying a unique molecular identifier (UMI) sequence as non-natural nucleic acid sequence and optionally an adaptor sequence as non-natural nucleic acid sequence, if present, in the aligned paired-end reads; (e) deleting said identified UMI sequence, if present, at the 5' end of each paired-end read, and optionally, if present, also at the 3' end of each paired-end read; (f) optionally deleting said identified adaptor sequence, if present, at the 3' end of each paired-end read; (g) inputting a truncated read obtained in step (e) or optionally in step (f) into a genomic sequence alignment in order to detect sequence differences vis-à-vis a reference sequence; (h) comparing identified sequence differences with a reference library of sequence differences linked to associated diseases; and (i) deducing the subject's health status and prognosis from the comparison result obtained in step (h).

The deletion of said non-natural nucleic acid sequences in the original paired-end reads in steps (e) and (f) and use of the obtained truncated reads in subsequent sequence data processing increases the accuracy of sequence data analysis, such as the comparison of identified sequence differences with a reference library of sequence differences.

Performing a "massively parallel nucleic acid sequencing" as mentioned herein relates to the performance of next generation sequencing approaches as mentioned above or as known to a skilled person, including future forms of this methodology. The sequencing may include the preparation of templates, the sequencing, as well as subsequent imaging and initial data analysis steps.

Preparation steps may, for example, include randomly breaking nucleic acids such as genomic DNA into smaller sizes and generating sequencing templates such as fragment templates. Spatially separated templates can, for example, be attached or immobilized at solid surfaces which allows for a sequencing reaction to be performed simultaneously. In typical examples, a library of nucleic acid fragments is generated. Subsequently, the fragments are denatured into single strands and captured by beads. After amplification and a possible enrichment a huge number of templates may be attached or immobilized in a polyacrylamide gel or be chemically crosslinked to an amino-coated glass surface or be deposited on individual titer plates. Alternatively, solid phase amplification may be employed. In this approach forward and reverse primers are typically attached to a solid support. The surface density of amplified fragments is defined by the ratio of the primers to the template on the support. This method may produce millions of spatially separated template clusters which can be hybridized to universal sequencing primers for sequencing reactions. Further suitable options include multiple displacement amplification methods.

Suitable sequencing methods include, but are not limited to, short read sequencing methods like cyclic reversible termination (CRT) or sequencing by synthesis (SBS) by Illumina. Further details with respect to the sequencing approach would be known to the skilled person or can be derived from suitable literature sources such as Goodwin et al., Nature Reviews Genetics, 2016, 17, 333-351, or van Dijk et al., Trends in Genetics, 2014, 9, 418-426.

A "subject's sample" as used herein may be any suitable sample derived from a subject. The sample may be derived from any patient or subject afflicted by a disease or condition. In certain embodiments, the sample is a tumor sample, i.e. the nucleic acids may be extracted from a tumor of a patient. In other embodiments, the sample may be a liquid biopsy sample derived from blood or urine or other body fluids. Also envisaged is to make use of previously deposited samples, e.g. samples derived from the umbilical cord.

The sample to be used is preferably a sample comprising one or more cells, e.g. premalignant or malignant cells, or any other type of cell which comprises a genotypic constitution associated with the disease. In certain embodiments the sample may comprise cells from a solid tumor or soft-tissue tumor or a metastatic lesion. Also envisaged is the use of a sample comprising tissue or cells from a surgical margin. Further envisaged is the employment of a histologically normal tissue obtained in a biopsy, e.g. as control. The present invention also relates to the use of one or more circulating tumor cells (CTC), e.g. obtained from blood samples. The sample may also be a sample comprising circulating tumor DNA (ctDNA). Further envisaged is the use of cell free DNA (cfDNA). Such DNA may, for example, be present in blood samples or processed blood samples, or other liquid samples obtained from a subject. Additionally, a blood, plasma or serum sample from the same subject having a tumor or being at risk of having a tumor may be used. Further, the sample may be a paraffin-embedded or FFPE-sample.

In certain embodiments, the in vitro method as mentioned above includes a preparation step for nucleic acids which comprises a hybrid-capture based nucleic acid enrichment for genomic regions of interest, i.e. targeted sequences such as exonic sequences etc. as defined above. The term "hybrid-capture based nucleic acid enrichment" as used herein, means that a library of nucleic acids is contacted with hybrid capture probes, either being in solution or being immobilized on a substrate, which substrate comprises a plurality of baits, e.g. oligonucleotide baits complementary to a gene or genomic region of interest to form a hybridization mixture; and subsequently, a plurality of bait/nucleic acid hybrids is separated from the mixture, e.g. by binding to an entity allowing for separation. This enriched mixture may subsequently be purified or further processed. The identity, amount, concentration, length, form etc. of the baits may be adjusted in accordance with the intended hybridization result. Thereby, focusing on a gene or region of interest may be achieved, since only those fragments or nucleic acids are capable of hybridizing which show complementarity to the bait sequence. The present invention envisages further variations and future developments of the above-mentioned approach. Further details are known to the skilled person or can be derived from suitable literature sources such as Mertes et al., 2011, Brief Funct Genomics, , 10(6), 374-386; Frampton et al., 2013, Nature Biotechnology, 31(11), 1023-1031; Gnirke et al., 2009, Nature Biotechnology, 27(2), 182-189 or from Teer et al, 2010, Genome Res, 20(10), 1420-1431.

Aligning and identification steps for UMI and optionally adaptor sequences of the method for diagnosing are similar to the steps as described herein above and may be implemented in the same manner as described above.

Subsequent to the deletion of the non-natural sequences (UMI and optionally adaptor sequences), the modified truncated reads are inputted into a genomic sequence alignment, e.g. as described herein above, in order to detect potential sequence differences vis-à-vis a reference sequence.

Identified sequence differences, if present, may be compared with a reference library of sequence differences linked to associated diseases. Such diseases may, for example, be cancer, an autoimmune disease, a renal disease, a cardiovascular disease or any other disease type which has a genotypic cause.

Advantageously, the deletion of said non-natural nucleic acid sequences in the reads as mentioned above increases the accuracy of the comparison and allows for a high-quality diagnostic approach.

Finally, the subject's health status may be determined, e.g. a specific disease may be identified. Accordingly, a therapy plan and a prognosis from the comparison result obtained in previous step may be obtained and presented to the subject.

In a further preferred embodiment, the method as described herein above comprises the additional step of providing a report on the obtained results as to the determination of a subject's health status as well as its use for the guidance of a treatment decision. Such a report may be provided in any suitable manner or form, e.g. as electronic file, as electronic file distributed or accessible over the internet, e.g. provided in cloud or deposited on a server, or web-based, e.g. provided on suitable web-site. Alternatively, the report may be provided in paper form. The report may be provided and thus drafted in a corresponding form to a patient (including information relevant for the patient), a relative or other person associated with the patient (including information relevant for this person), a caregiver (including information relevant for the caregiver), a physician (including information relevant for the physician), an oncologist (including information relevant for the oncologist), or a hospital or clinic (including information relevant for the institution), or third party payors, insurance companies or government offices (including information relevant for these entities). The report may accordingly be redacted, modified, extended or adjusted to the above specified recipient. For example, information relevant for the oncologist, e.g. as to the copy number value, may be omitted in the report for the patient etc.

Among the elements the report may comprise, the present invention envisages one or more of the following:
(i) An output from the method performed, which may include the determined sequence difference if present (this information may be relevant for the physician, hospital and possibly also insurance companies).
(ii) Information on the comparison results wherein said information comprises information on prognosis and/or potential or suggested therapeutic options. The corresponding information may also comprise information on prognosis of the disease, and/or on potential or suggested therapeutic options. Also included may be a conclusion on the most promising treatment or a potential therapy plan. The corresponding information may be derived from suitable databases or literature sources, e.g. by a medical professional. These sources may also be provided in the report.
(iii) Further included may be information on the likely effectiveness of a therapeutic option or on the acceptability of a therapeutic option. Moreover, information on the advisability of applying the therapeutic option to a patient having a sequence modification in the report may be given. The corresponding information may be derived from suitable databases or literature sources. These sources may also be provided in the report.
(iv) Also included may be information or a recommendation on the administration of a specific drug or compound, as well as the details on potential administration schemes, administration routes, dosage regimen, treatment regimen etc. This may further be extended to the potential administration of additional drugs, e.g. if this information about a patient is already known or if a co-administration of drugs is necessary or advisable.

In further embodiments, the present invention also envisages a determination system which performs any of the herein above defined methods. The system may be implemented on any suitable storage or computer platform, e.g. be cloud-based, internet-based, intra-net based or present on local computer or mobile devices, such as cellphones etc.

In a further set of embodiments, the present invention envisages the provision of a data processing apparatus or system comprising means for carrying out any one or more steps of the methods of the present invention as mentioned herein above.

In further embodiments, the present invention additionally envisages a computer program product which performs any of the herein above defined methods or any one or more steps of the methods of the present invention as mentioned herein above.

Also envisaged is the provision of a computer-readable storage medium comprising a computer program product as defined above. The computer-readable storage medium may be connected to a server element or be present in a cloud structure or be connected via internet to one or more database structures or client databases etc.

The figures are provided for illustrative purposes. It is thus understood that the figures are not to be construed as limiting. The skilled person in the art will clearly be able to envisage further modifications of the principles laid out herein.

## Claims

1. A method for eliminating non-natural nucleic acid sequence portions from paired-end reads of nucleic acid fragments comprising:
(a) providing paired-end reads of nucleic acid fragments, wherein one of the two paired-end reads that constitute a read pair of a nucleic acid fragment is converted into its reverse complement form;
(b) aligning the two paired-end reads of a read pair of step (a) with each other;
(c) identifying overlapping sequence regions in the aligned paired-end reads;
(d) identifying a unique molecular identifier (UMI) sequence as non-natural nucleic acid sequence in the aligned paired-end reads;
(e) optionally storing said identified UMI sequence;
(f) deleting said identified UMI sequence, if present, at the 5' end of each paired-end read; and
(g) deleting said identified UMI sequence, if present, at the 3' end of each paired-end read.

2. The method of claim 1, wherein said identification of the overlap in the aligned paired-end reads comprises the steps of:
(i) sliding one paired-end read past the other paired-end read over the entire length of the paired-end reads and determining for each position whether an overlap exists; and
(ii) selecting the sliding position which provides the maximum number of overlapping bases.

3. The method of claim 2, wherein said step (ii) of selecting the sliding position of the maximum number of overlapping bases is performed only if the overall number of mismatches between the paired-end reads is below 5% to 15% of all bases.

4. The method of claim 2, wherein said step (ii) of selecting the sliding position of the maximum number of overlapping bases is performed only if the likelihood of correct overlapping is above 90%, 95%, or 99%.

5. The method of claim 2, wherein said step (ii) of selecting the sliding position of the maximum number of overlapping bases is performed only if a minimum number of overlapping bases is reached, wherein said minimum number is 10, 15, 20, 25 or 30.

6. The method of any one of claims 1 to 5, wherein said identification of a UMI sequence in the aligned paired-end reads uses recorded information on UMI sequences and/or standardized or conventional position and length information.

7. The method of any one of claims 1 to 6, wherein said identification of a UMI sequence is an identification of a 3' UMI sequence of one paired-end read and wherein said identification requires the identification of an overlapping 5' UMI sequence in the other paired-end read of the read pair.

8. The method of any one of claims 1 to 7, additionally comprising as step (d-1) identifying an adaptor sequence as non-natural nucleic acid sequence, if present, in the aligned paired-end reads; and as step (f-1) deleting said identified adaptor sequence at the 3' end of each paired-end read.

9. The method of claim 8, wherein said adaptor sequence is identified in the 3'-terminus of each paired-end read of the aligned paired-end reads.

10. The method of claim 9, wherein said adaptor sequence is identified as being located 3' of a UMI sequence and wherein both paired-end reads comprise UMI sequences completely overlapping in the alignedpaired-end reads.

11. The method of claim 8, wherein said deletion of said identified adaptor sequence is performed concomitant with the deletion of said 3' UMI sequence.

12. The method of any one of claims 1 to 11, wherein said paired-end reads of a read pair have a length of 10 to 10.000 bases.

13. An in vitro method for diagnosing a subject, comprising:
(a) performing a massively parallel nucleic acid sequencing of nucleic acids extracted from a subject's sample, preferably a tumor biopsy sample or a liquid biopsy sample, to obtain paired-end reads, wherein one of the two paired-end reads that constitute a read pair of a nucleic acid fragment is converted into its reverse complement form;
(b) aligning the two paired-end reads of a read pair obtained in step (a) with each other;
(c) identifying overlapping sequence regions in the aligned paired-end reads;
(d) identifying a unique molecular identifier (UMI) sequence as non-natural nucleic acid sequence and optionally an adaptor sequence as non-natural nucleic acid sequence, if present, in the aligned paired-end reads;
(e) deleting said identified UMI sequence, if present, at the 5' end of each paired-end read, and optionally, if present, also at the 3' end of each paired-end read;
(f) optionally deleting said identified adaptor sequence, if present, at the 3' end of each paired-end read;
(g) inputting a truncated read obtained in step
(e) and optionally in step (f) into a genomic sequence alignment in order to detect sequence differences vis-à-vis a reference sequence;
(h) comparing identified sequence differences with a reference library of sequence differences linked to associated diseases; and
(i) deducing the subject's health status and prognosis from the comparison result obtained in step (h) .

14. The method of claim 13, wherein the method further comprises providing a report in electronic, web-based, or paper form to a subject or to another person or entity, a caregiver, a physician, an oncologist, a hospital, a clinic, a third-party payor, an insurance company or a government office.

15. The method of claim 14, wherein the report comprises one or more of:
(i) output from the method, comprising the determined sequence difference, if present;
(ii) information on the meaning of the comparison results wherein said information comprises information on prognosis and/or potential or suggested therapeutic options;
(iii) information on the likely effectiveness of a therapeutic option, the acceptability of a therapeutic option, or the advisability of applying the therapeutic option to a subject having a sequence modification; or
(iv) information or a recommendation on the administration of a drug, the administration at a preselected dosage, or in a preselected treatment regimen in combination with other drugs, to the subject.
